# EUROPEAN PATENT SPECIFICATION

(11) **EP 2 168 620 B1**
(45) Date of publication and mention of the grant of the patent: **20.07.2011**
(21) Application number: 09171803.1
(22) Date of filing: 30.09.2009
(51) Int. Cl.: A61M 5/315

(54) **Diluent/Medication Mixing Syringe Assembly**
Spritzenanordnung für eine Verdünnungs- / Arzneimittelmischung
Ensemble de seringue à mélange de diluant/médicaments

(30) Priority: 30.09.2008 US 101410 P
(43) Date of publication of application: 31.03.2010
(73) Proprietor: Tyco Healthcare Group LP, Mansfield, MA 02048 (US)
(72) Inventor: Chebator, Casey, Weymouth, MA 02093 (US); Braga, Richard, North Easton, MA 02356 (US)
(74) Representative: Gray, James

(56) References cited:
- WO-A1-99/17820
- US-A- 3 164 303
- US-A1- 2006 079 834

## Description

### BACKGROUND

The present disclosure is directed generally to an interface device for use with standard luer lock syringes. More particularly, the present disclosure is directed to an interface device capable of interfacing with standard luer lock syringes to sequentially deliver multiple fluids from the device to a medical access device. A syringe assembly as defined in the preamble of claim 1 is disclosed in US 2006/0079834.

Numerous techniques are employed for the administration of "medical liquids" (e.g. liquid medication and flush/lock solutions) to a patient. In particular, where repeated medication infusions are required, medical liquids are often administered via the use of a central venous access catheter (CVAD) that is fluidly interconnected or interconnectable to one or more medical liquid sources via an associated tubing line set. Typically, the CVAD is inserted into the vein of a patient and left there for multiple intravenous (IV) infusions during an extended course of medication therapy.

It is usual practice to purge the CVAD with a flush solution (e.g. a saline solution) prior to and at the completion of a given liquid medication infusion. Pre-infusion flushing verifies that the vascular catheter is primed and clear of obstructions. Post infusion flushing/locking not only flushes through any remaining liquid medication to achieve the desired therapeutic effect, but also reduces the risk of CVAD blockage.

Typically, post infusion flushing/locking procedures involve a two step procedure wherein in a first step, a flush solution, e.g., saline, is infused into a CVAD to flush any medication remaining in the CVAD from the CVAD into a patient, and in a second step, a lock solution, e.g., heparin, is infused into the CVAD to prevent clotting of the CVAD. The lock solution may also include anti-microbial agents to reduce the risk of infection to the patient from bacterial colonization of the CVAD.

A number of approaches are currently utilized to perform flushing/locking procedures including the use of multiple prefilled syringes, which subjects the CVAD to multiple connections thus, increasing the risk of patient exposure to contaminants.

It would be desirable to provide a device which could interface with a standard luer-lock syringe to facilitate performance of flushing/locking procedures using only a single connection to the CVAD.

### SUMMARY

According to the present invention there is provided a diluent/medication mixing syringe assembly comprising:
a syringe body having an open proximal end and a fluid outlet on a distal end;
a plunger assembly including a plunger body and a plunger head supported on a distal end of the plunger body, the plunger head slidably and sealingly engaging an inner wall of the syringe body, the plunger head and syringe body defining a reservoir in the distal end of the syringe body; and
a cannula supported on and extending through the plunger assembly, the cannula defining a longitudinal channel which extends through the plunger assembly into the reservoir, wherein the plunger body and the plunger head define a longitudinal bore which is dimensioned to receive the cannula, characterized in that the cannula is slidably positioned within the longitudinal bore such that the distal end of the cannula is movable axially within the reservoir.

The cannula may be rotatably positioned within the longitudinal bore. The syringe assembly may further include a mixing element supported on the distal end of the cannula. The mixing element may include at least one orifice which extends through the mixing element, the at least one orifice being configured to cause turbulence within the reservoir when the cannula is moved axially.

The at least one orifice may include a plurality of orifices. The proximal end of the cannula may include an adaptor which is configured to releasably engage a supply of fluid. The adaptor may be a female luer type connector. The syringe assembly may further include a sealing member positioned within the longitudinal bore of the plunger body about the cannula.

The sealing member may be formed of a resilient material which is configured to seal the longitudinal bore upon removal of at least a proximal portion of the cannula from the longitudinal bore. The cannula may include a weakened portion which facilitates separation of the proximal portion of the cannula from a distal portion of the cannula upon application of a predetermined force on the cannula. The predetermined force may be from about 22N (two pounds) to about 36N (five pounds). The syringe assembly may further include a male luer connector positioned on the distal end of the syringe body about the fluid outlet.

### Brief Description Of The Drawings

Various embodiments of the presently disclosed device for interfacing with standard luer lock syringes ("interface device") are disclosed herein with reference to the drawings, wherein:
FIG. 1 is a side perspective view from the distal end of one embodiment of the presently disclosed interface device with a standard syringe assembly received therein;
FIG. 2 is a side perspective exploded view of the interface device and syringe assembly shown in FIG. 1:
FIG. 3 is a view of the syringe assembly;
FIG. 4 is a view of the syringe assembly;
FIG. 5 is a side cross-sectional view of the syringe assembly ;
FIG. 6 is a side cross-sectional view of the syringe assembly; and
FIG. 7 is a side cross-sectional view of the presently disclosed syringe assembly.

### DETAILED DESCRIPTION OF EMBODIMENTS

Embodiments of a presently disclosed interface device not within scope of the claims will now be described in detail with reference to the drawings wherein like reference numerals designate identical or corresponding elements in each of the several views. In this description, the term proximal is generally used to indicate the relative nearness of a referenced item to a user of the device and the term distal is used to indicate the relative remoteness of a referenced item to a user of the device.

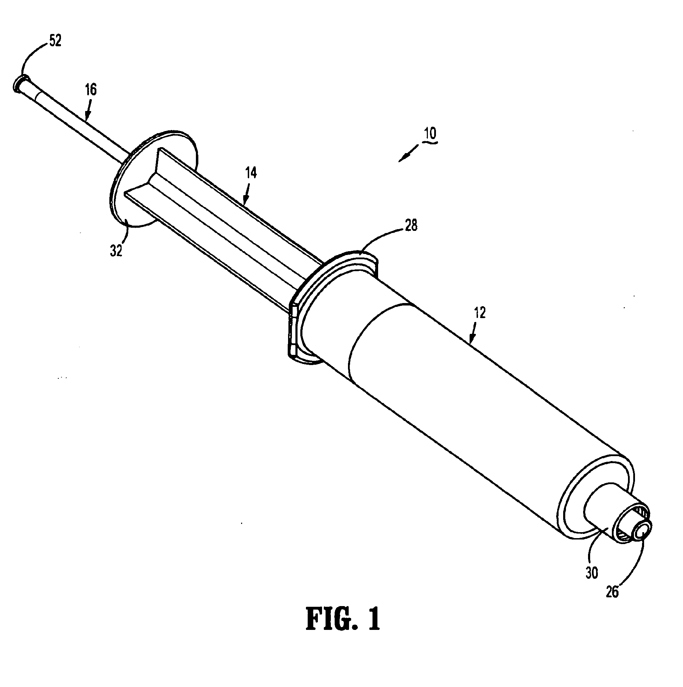

FIGS. 1-3 illustrate one embodiment of the presently disclosed device, shown generally as 10. Syringe assembly is known in the medical art and will only be described in detail necessary to facilitate description of device 10.

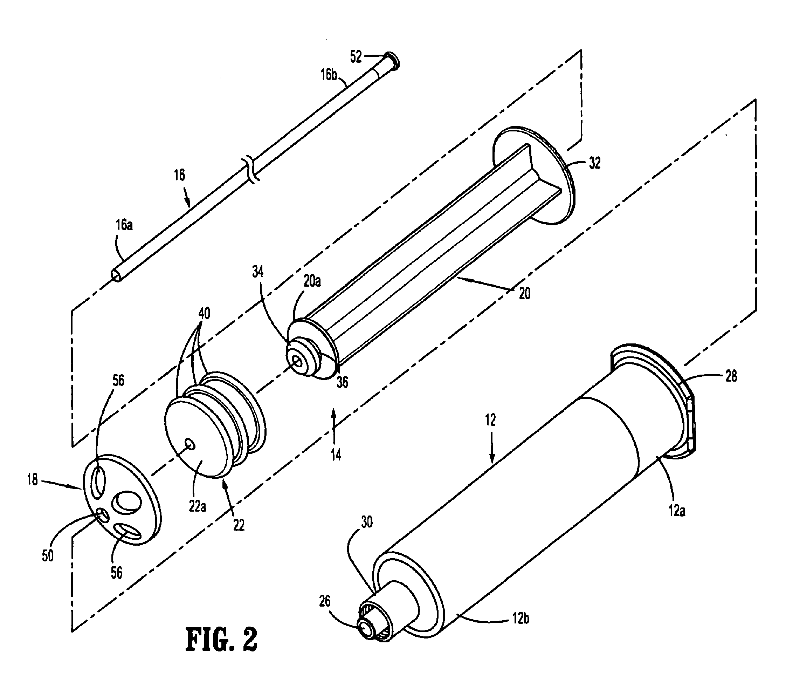

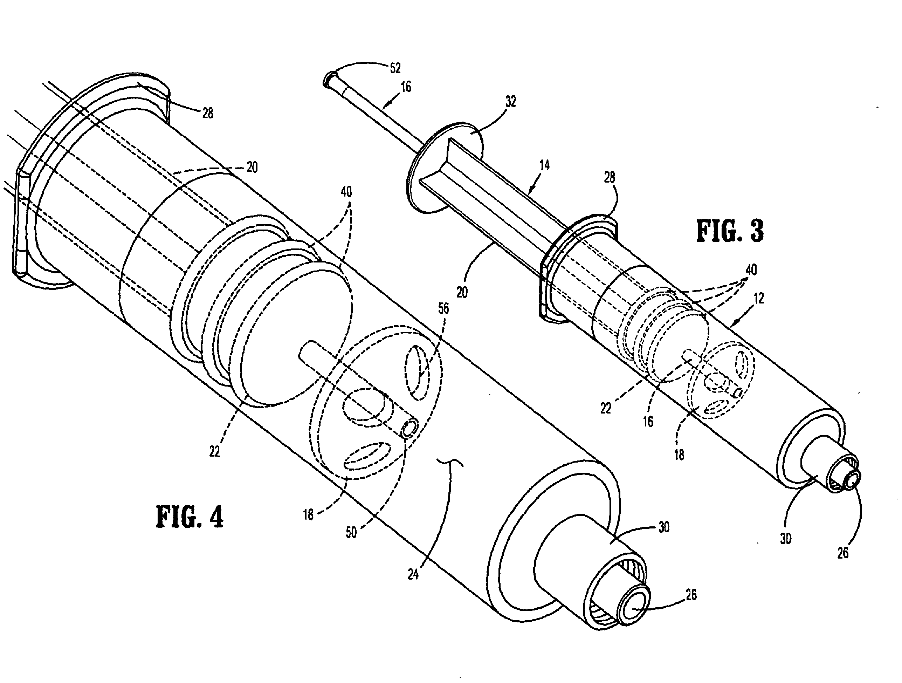

Referring to FIGS. 1-2 (not within scope of the claims), interface device 10 includes a body 12 including a proximal body portion 14, a distal body half-section and a distal body half-section. Proximal portion 14 includes a proximal gripping flange 13 and defines a cylindrical bore 20 (FIG. 2A). Each distal half-section 16 and 18 defines a semi-annular groove 21 and a longitudinal groove 22. Distal half-section 16 further defines a channel 24 (FIGS. 3 and 4) which extends from within semi-annular groove 21 and intersects longitudinal groove 22 at a location proximal to a distal end of longitudinal groove 22. The distal end of each distal half-section 16 and 18 further defines connector half-sections 25.

Referring to FIGS. 3 and 4 (not within scope of the claims), distal half-sections 16 and 18 are secured to a distal end of proximal portion 14 of body 12 and to each other using a known fastening technique, e.g., sonic welding, adhesives, etc., such that semi-annular grooves 21 define an annular groove 30, longitudinal grooves 22 define an outlet channel 32 and connector half-sections 25 define a male luer-type connector 34 or similar structure.

Referring to FIGS. 2-3 (not within scope of the claims), a plunger 40 is slidably positioned within cylindrical bore 20 of body 12. Plunger 40 is substantially cylindrical and is dimensioned to slidably and sealingly engage an inner wall 12a of body 12. Plunger 40 defines an annular channel 42 about its sidewall 43 and a channel 44 which extends from a top surface 46 of plunger 40 to annular channel 42. A bottom surface 46a of plunger 40 defines a first fluid reservoir 47 with the distal end of body 12. Top surface 46 of plunger 40 includes a luer-type connector 48 positioned about an inlet end of channel 44. Connector 48 is dimensioned to be engaged by a corresponding luer-type connector on the distal end of a standard syringe assembly 100 in a fluid tight manner as will be discussed in further detail below. Referring to FIG. 3. syringe assembly 100 includes a syringe body 102 and a plunger assembly 104. Syringe body 102 is dimensioned to be tightly received within cylindrical bore 20 of body 12 of interface device 10. Plunger assembly 104 includes a plunger rod 106 and a plunger head 108 which is secured to a distal end of plunger rod 106 and is slidably positioned within syringe body 102. Syringe body 102 and a distal face of plunger head 108 define a fluid reservoir 110. A distal end of syringe body 102 defines a fluid outlet 112 and a luer-type connector 113.

Referring to FIGS. 3-6 (not within scope of the claims), in use a standard syringe assembly 100 is positioned within cylindrical bore 20 of interface device 10 such that fluid outlet 112 is received within channel 44 of plunger 40 and connector 48 of plunger 40 engages connector 113 of syringe 100. (FIG. 4). Although not shown, a cap can be secured to connector 34 on the distal end of interface device 10 to seal outlet channel 32 until immediately prior to use of interface device 10.

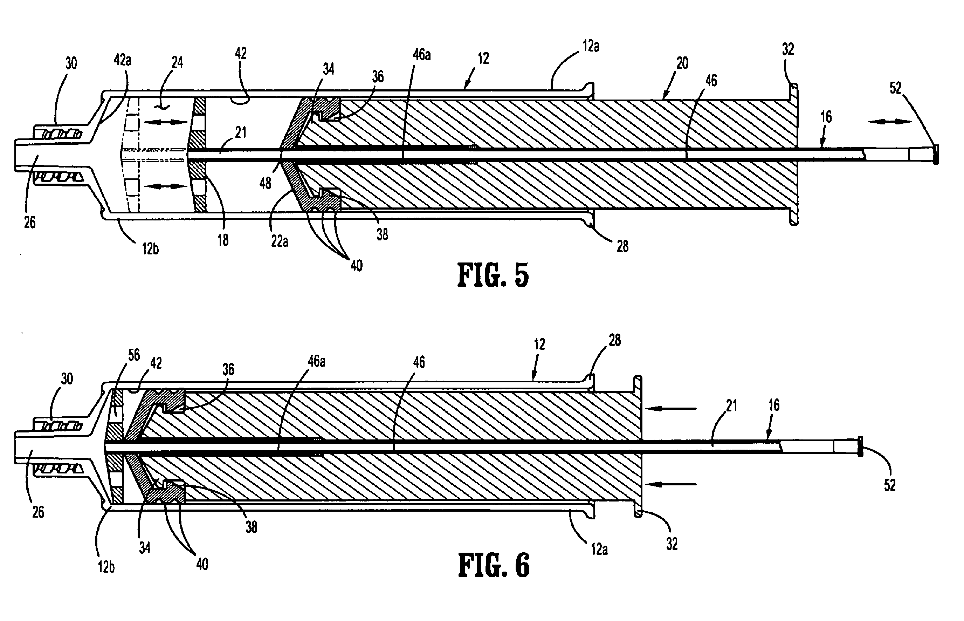

Referring to FIG. 5 (not within scope of the claims), after syringe assembly 100 has been positioned within cylindrical bore 20 of body 12, the cap (not shown) positioned over outlet channel 32 can be removed and connector 34 of interface device 10 can be secured to a central venous access device (not shown) or the like. Next, syringe body 102 which includes a gripping flange 114 can be pressed into cylindrical bore 20 of body 12 in the direction indicated by arrows "A" in FIG. 5. When syringe body 102 moves into cylindrical bore 20 of body 12, plunger 40 is forced from a retracted position (FIG. 4) to an advanced position (FIG. 5) to dispense a first fluid from reservoir 47 of interface device 10 via fluid outlet channel 32. As plunger 40 moves from the retracted position towards the advanced position, annular channel 42 and channel 44 which communicate with reservoir 110 within syringe body 102 are sealed by the inner wall 20a defining cylindrical bore 20. Thus, a second fluid within reservoir 110 of syringe body 102 is prevented from being dispensed from reservoir 110.

Referring again to FIG. 5 (not within scope of the claims), when plunger 40 nears the advanced position, annular channel 42 communicates with annular groove 30 of body 12. When channel 42 communicates with annular groove 30, reservoir 110 of syringe assembly 100 is in communication with outlet channel 32 via channel 44, annular channel 42, annular groove 30 and channel 24. It is also envisioned that the use of either annular channel 42 or annular groove 30 in the absence of the other would be capable of establishing communication between channel 44 and channel 24.

Referring to FIG. 6 (not within scope of the claims), when plunger 40 is in the advanced position and plunger assembly 104 is moved from its retracted position (FIG. 5) to its advanced position in the direction indicated by arrow "B" in FIG. 6, the second fluid located within reservoir 110 of syringe body 102 is forced from reservoir 110 through fluid outlet 112 of syringe body 102. Fluid flowing from outlet 112 flows through channel 44 of plunger 40 and channel 24 of body 12 of interface device 10, and through fluid outlet 32 of device 10.

In one embodiment (not within scope of the claims), the first fluid within reservoir 47 of device 10 is a flush solution such as saline and the second fluid within reservoir 110 of syringe body 102 is a lock solution such as heparin. As discussed above, a flush solution is used to flush medication remaining in a catheter from the catheter into a patient whereas a lock solution, such as heparin, is used to maintain the patency of a catheter lumen while minimizing the risk of infection to a patient. Although the use of interface device 10 has been described for use with a flush solution and a standard syringe assembly having a lock solution, it is envisioned that other fluids can be dispensed sequentially with the presently disclosed interface device and standard syringe assembly. It is also envisioned that reservoir 47 may be prefilled with the first fluid during manufacture of device 10.

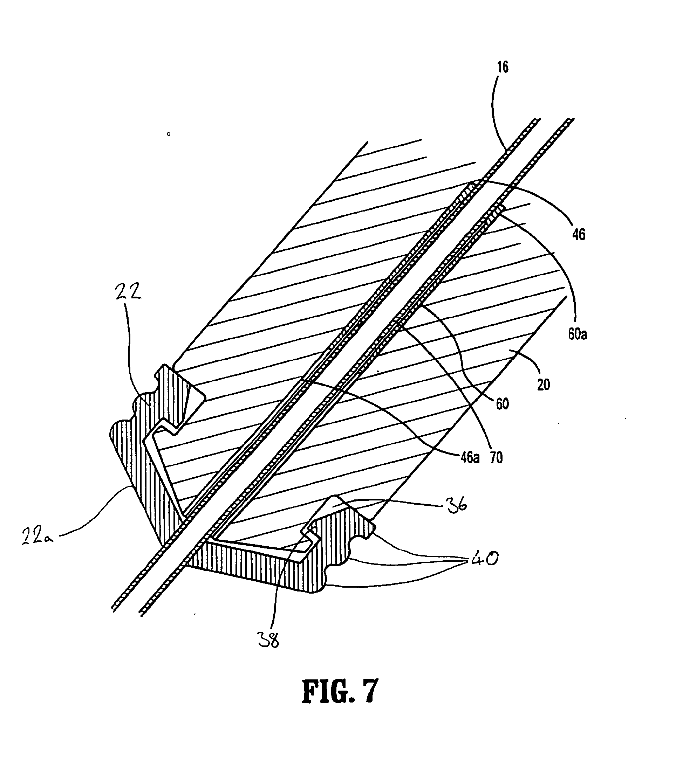

FIG. 7 (not within scope of the claims) illustrates an alternative embodiment of the presently disclosed interface device shown generally as 200. Interface device 200 is substantially similar to interface device 10 but includes a body 212 defining two cylindrical bores 220a and 220b and a plunger 240a and 240b positioned within each cylindrical bore 220a and 220b, respectively. Each cylindrical bore 220a and 220b is dimensioned to receive a syringe assembly 100 as described above. Plungers 240a and 240b are identical to plunger 40 (FIG. 5) described above and will not be described in further detail herein. Interface device 200 in association with two syringe assemblies 100 functions to inject four fluids sequentially into a catheter (not shown) in the manner described above with respect to interface device 10.

It will be understood that various modifications may be made to the embodiments disclosed herein. For example, the interface device may include three or more cylindrical bores and plungers and/or consist of single piece construction. Therefore the above description should not be construed as limiting, but merely as exemplifications of embodiments. Those skilled in the art will envision other modifications within the scope of the claims appended hereto.

## Claims

1. A diluent/medication mixing syringe assembly (10) comprising:
a syringe body (12) having an open proximal end (12a) and a fluid outlet (26) on a distal end (126);
a plunger assembly (14) including a plunger body (20) and a plunger head (22) supported on a distal end (20a) of the plunger body (20), the plunger head (22) slidably and sealingly engaging an inner wall (42) of the syringe body (12), the plunger head (22) and syringe body (12) defining a reservoir (24) in the distal end (126) of the syringe body (12); and
a cannula (16) supported on and extending through the plunger assembly (14), the cannula (16) defining a longitudinal channel (21) which extends through the plunger assembly (14) into the reservoir (24), wherein the plunger body (20) and the plunger head (22) define a longitudinal bore (46,48) which is dimensioned to receive the cannula (16), **characterized in that** the cannula (16) is slidably positioned within the longitudinal bore (46) such that the distal end (16a) of the cannula (16) is movable axially within the reservoir (24).

2. The syringe assembly (10) according to claim 1, wherein the cannula (16) is rotatably positioned within the longitudinal bore (46,48).

3. The syringe assembly (10) according to any one of the preceding claims, further including a mixing element (18) supported on the distal end (16a) of the cannula (16).

4. The syringe assembly (10) according to claim 3, wherein the mixing element (18) includes at least one orifice (56) which extends through the mixing element (18), the at least one orifice (56) being configured to cause turbulence within the reservoir (24) when the cannula (16) is moved axially.

5. The syringe assembly (10) according to claim 4, wherein the at least one orifice (56) includes a plurality of orifices.

6. The syringe assembly (10) according to any one of the preceding claims, wherein the proximal end (16b) of the cannula (16) includes an adaptor (52) which is configured to releasably engage a supply of fluid.

7. The syringe assembly (10) according to claim 6, wherein the adaptor (52) is a female luer type connector.

8. The syringe assembly (10) according to claim 1 or claim 2, further including a sealing member (60) positioned within the longitudinal bore (46) of the plunger body (20) about the cannula (16).

9. The syringe assembly (10) according to claim 8, wherein the sealing member (60) is formed of a resilient material which is configured to seal the longitudinal bore (46) upon removal of at least a proximal portion (16b) of the cannula (16) from the longitudinal bore (46).

10. The syringe assembly (10) according to any one of the preceding claims, wherein the cannula (16) includes a weakened portion (70) which facilitates separation of the proximal portion (16b) of the cannula from a distal portion (16a) of the cannula (16) upon application of a predetermined force on the cannula (16).

11. The syringe assembly (10) according to claim 10, wherein the predetermined force is from about 22N (two pounds) to about 36N (five pounds).

12. The syringe assembly (10) according to any one of the preceding claims, further including a male luer connector (30) positioned on the distal end of the syringe body (12) about the fluid outlet (26).

## Patentansprüche

1. Spritzenanordnung (10) zum Mischen von Verdünnungsmittel/Arzneimittel, die Folgendes umfasst:
einen Spritzenkörper (12) mit einem offenen proximalen Ende (12a) und einem Flüssigkeitsauslass (26) an einem distalen Ende (126);
eine Kolbenanordnung (14) mit einem Kolbenkörper (20) und einem Kolbenkopf (22), der auf einem distalen Ende (20a) des Kolbenkörpers (20) gestützt wird, wobei der Kolbenkopf (22) gleitend und dichtend in eine Innenwand (42) des Spritzenkörpers (12) eingreift, wobei der Kolbenkopf (22) und der Spritzenkörper (12) ein Reservoir (24) im distalen Ende (126) des Spritzenkörpers (12) definieren; und
eine Kanüle (16), die auf der Kolbenanordnung (14) gestützt wird und sich durch diese erstreckt, wobei die Kanüle (16) einen Längskanal (21) definiert, der sich durch die Kolbenanordnung (14) bis in das Reservoir (24) erstreckt, worin der Kolbenkörper (20) und der Kolbenkopf (22) eine Längsbohrung (46, 48) definieren, die so bemessen ist, dass sie die Kanüle (16) aufnimmt, **dadurch gekennzeichnet, dass** die Kanüle (16) gleitend in der Längsbohrung (46) angeordnet ist, so dass das distale Ende (16a) der Kanüle (16) axial im Reservoir (24) bewegt werden kann.

2. Spritzenanordnung (10) nach Anspruch 1, worin die Kanüle (16) drehbar in der Längsbohrung (46, 48) angeordnet ist.

3. Spritzenanordnung (10) nach einem der vorhergehenden Ansprüche, die ferner ein auf dem distalen Ende (16a) der Kanüle (16) gestütztes Mischelement (18) aufweist.

4. Spritzenanordnung (10) nach Anspruch 3, worin das Mischelement (18) zumindest eine Öffnung (56) aufweist, die sich durch das Mischelement (18) erstreckt, wobei die zumindest eine Öffnung (56) so konfiguriert ist, dass sie Turbulenz im Reservoir (24) verursacht, wenn die Kanüle (16) axial bewegt wird.

5. Spritzenanordnung (10) nach Anspruch 4, worin die zumindest eine Öffnung (56) eine Vielzahl von Öffnungen aufweist.

6. Spritzenanordnung (10) nach einem der vorhergehenden Ansprüche, worin das proximale Ende (16b) der Kanüle (16) einen Adapter (52) aufweist, der so konfiguriert ist, dass er lösbar in einen Flüssigkeitsvorrat eingreift.

7. Spritzenanordnung (10) nach Anspruch 6, worin der Adapter (52) ein Aufnahmeverbinder vom Luer-Typ ist.

8. Spritzenanordnung (10) nach Anspruch 1 oder Anspruch 2, die ferner ein Dichtungselement (60) aufweist, das in der Längsbohrung (46) des Kolbenkörpers (20) um die Kanüle (16) angeordnet ist.

9. Spritzenanordnung (10) nach Anspruch 8, worin das Dichtungselement (60) aus einem nachgiebigen Material besteht, das so konfiguriert ist, dass es die Längsbohrung (46) bei Entfernung zumindest eines proximalen Abschnitts (16b) der Kanüle (16) von der Längsbohrung (46) abdichtet.

10. Spritzenanordnung (10) nach einem der vorhergehenden Ansprüche, worin die Kanüle (16) einen geschwächten Abschnitt (70) aufweist, der die Trennung des proximalen Abschnitts (16b) der Kanüle von einem distalen Abschnitt (16a) der Kanüle (16) bei Aufbringen einer vorbestimmten Kraft auf die Kanüle (16) erleichtert.

11. Spritzenanordnung (10) nach Anspruch 10, worin die vorbestimmte Kraft ca. 22N (zwei Pfund) bis ca. 36N (fünf Pfund) beträgt.

12. Spritzenanordnung (10) nach einem der vorhergehenden Ansprüche, die ferner einen Luer-Steckverbinder (30) aufweist, der am distalen Ende des Spritzenkörpers (12) um den Flüssigkeitsauslass (26) angeordnet ist.

## Revendications

1. Ensemble formant seringue de mélange diluant/médicament (10) comprenant :
un corps de seringue (12) ayant une extrémité proximale ouverte (12a) et une sortie de fluide (26) sur une extrémité distale (126) ;
un ensemble de plongeur (14) comprenant un corps de plongeur (20) et une tête de plongeur (22) supportée sur une extrémité distale (20a) du corps de plongeur (20), la tête de plongeur (22) engageant en coulissement et de manière étanche une paroi interne (42) du corps de seringue (12), la tête de plongeur (22) et le corps de seringue (12) définissant un réservoir (24) dans l'extrémité distale (126) du corps de seringue (12) ; et
une canule (16) supportée sur, et s'étendant à travers, l'ensemble de plongeur (14), la canule (16) définissant un canal longitudinal (21) qui s'étend à travers l'ensemble de plongeur (14) jusque dans le réservoir (24), où le corps de plongeur (20) et la tête de plongeur (22) définissent un alésage longitudinal (46, 48) qui est dimensionné pour recevoir la canule (16), **caractérisé en ce que** la canule (16) est positionnée en coulissement au sein de l'alésage longitudinal (46) de manière que l'extrémité distale (16a) de la canule (16) soit mobile axialement au sein du réservoir (24).

2. Ensemble formant seringue (10) selon la revendication 1, dans lequel la canule (16) est positionnée de manière à tourner au sein de l'alésage longitudinal (46, 48).

3. Ensemble formant seringue (10) selon l'une quelconque des revendications précédentes, comprenant, en outre, un élément de mélange (18) supporté sur l'extrémité distale (16a) de la canule (16).

4. Ensemble formant seringue (10) selon la revendication 3, dans lequel l'élément de mélange (18) comprend au moins un orifice (56) qui s'étend à travers l'élément de mélange (18), l'au moins un orifice (56) étant configuré pour provoquer une turbulence au sein du réservoir (24) lorsque la canule (16) est déplacée axialement.

5. Ensemble formant seringue (10) selon la revendication 4, dans lequel l'au moins un orifice (56) comprend une pluralité d'orifices.

6. Ensemble formant seringue (10) selon l'une quelconque des revendications précédentes, dans lequel l'extrémité proximale (16b) de la canule (16) comprend un adaptateur (52) qui est configuré pour engager de manière libérable une source de fluide.

7. Ensemble formant seringue (10) selon la revendication 6, dans lequel l'adaptateur (52) est un raccord femelle de type Luer.

8. Ensemble formant seringue (10) selon la revendication 1 ou la revendication 2, comprenant, en outre, un élément d'étanchéité (60) positionné au sein de l'alésage longitudinal (46) du corps de plongeur (20) autour de la canule (16).

9. Ensemble formant seringue (10) selon la revendication 8, dans lequel l'élément d'étanchéité (60) est formé d'un matériau élastique qui est configuré pour étancher l'alésage longitudinal (46) lors du retrait, de l'alésage longitudinal (46), d'au moins une partie proximale (16b) de la canule (16).

10. Ensemble formant seringue (10) selon l'une quelconque des revendications précédentes, dans lequel la canule (16) comprend une partie affaiblie (70) qui facilite la séparation de la partie proximale (16b) de la canule (16) d'une partie distale (16a) de la canule (16) lors de l'application d'une force prédéterminée sur la canule (16).

11. Ensemble formant seringue (10) selon la revendication 10, dans lequel la force prédéterminée est d'environ 22 N (deux livres) à environ 36 N (cinq livres).

12. Ensemble formant seringue (10) selon l'une quelconque des revendications précédentes, comprenant, en outre, un raccord mâle de type Luer (30) positionné sur l'extrémité distale du corps de seringue (12) autour de la sortie de fluide (26).
